# EUROPEAN PATENT APPLICATION

(11) **EP 2 957 319 A1**
(43) Date of publication of application: **23.12.2015**
(21) Application number: 13875316.5
(22) Date of filing: 18.02.2013
(51) Int. Cl.: A61N 1/30

(54) **MEDICINE ADMINISTERING DEVICE, MEDICINE ADMINISTERING SYSTEM, AND CONTROL METHOD FOR SAME**

(71) Applicant: Terumo Kabushiki Kaisha, Tokyo 151-0072 (JP)
(72) Inventor: NARIMATSU, Kiyoyuki, Fujinomiya-shi Shizuoka 418-0015 (JP)
(74) Representative: Casalonga
(86) International application number: PCT/JP2013/000882
(87) International publication number: WO 2014/125518

(57) **Abstract**

A drug administration device, which applies a voltage between electrodes so as to percutaneously administer a drug, determines a sharing administration rate so that an administration rate of the set drug is shared and realized between the drug administration device and another drug administration device, and transmits the administration rate shared with the other drug administration device, to the other drug administration device. When the drug administration device receives the administration rate from the other drug administration device and one device between the drug administration device and the other drug administration device can no longer maintain the determined administration rate, the drug administration device changes the administration rate of the other device so as to compensate for an insufficient administration rate.

## Description

### Technical Field

The present invention relates to a drug administration device which percutaneously administers a drug, a drug administration system, and a control method thereof.

### Background Art

In general, oral administration or injection administration is widely used in order to administer a drug. In a case of oral administration, the drug is conveniently and very safely taken, but there is a problem in that a water-soluble drug or a polymeric drug cannot be sufficiently absorbed. In addition, in a case of injection administration, there is an advantage in that the drug works quickly, but there is an adverse effect in that a patient has to feel pain.

Currently, in order to compensate for the problems or adverse effects in these methods used in the related art, a drug administration system (hereinafter, referred to as a percutaneous drug administration system) through skin has been progressively developed. As the percutaneous drug administration system, several methods such as methods of using electrical energy, methods of using ultrasound waves, and the like have been proposed.

Among the methods of using electrical energy, iontophoresis is known as a partially commercialized method which has been studied for a long time. The iontophoresis is a method in which a charged drug is moved and absorbed in a site of a keratinous layer on the principle of electrophoresis, by placing positive and negative electrodes on two separated points of skin, and by generating a current which crosses the keratinous layer of the skin and then passes through an internal body side from the keratinous layer so that the current is connected from the positive electrode to the negative electrode. In principle, the charged drug is subject to promoted absorption, but the current also causes water to flow therein. For this reason, it has been reported that a drug having no charge or a drug having high molecular weight is also able to have improved skin permeability.

As an example of the iontophoresis, PTL 1 discloses that a patch having an electrode containing a drug is combined with a controller driven by a battery so as to supply power from the controller to the electrode. The controller disclosed in PTL 1 is small since the controller does not need to set a complicated profile and is used simply for switching currents to be supplied. Moreover, since the controller is driven by the battery, there is no possibility of restricting a patient to whom the drug is administered.

In addition, PTL 2 discloses a configuration in which a controller combined with a patch and a dedicated cradle are provided so that the dedicated cradle supplies the controller with a profile (current value setting or the like) to be set depending on the patch, by wireless communication. According to PTL 2, it is possible to perform more complicated current value control by using an easy operation while maintaining design features of a device.

### Citation List

### Patent Literature

[PTL 1] JP-T-11-506368
[PTL 2] JP-A-2010-172475

### Summary of Invention

### Technical Problem

Incidentally, according to the portable iontophoresis methods, as disclosed in PTL 1 or PTL 2, drug administration is performed at an administration rate designated in accordance with a supply current or a profile which is set for each drug administration device. The iontophoresis has an anode and a cathode in a pair, and a current is caused to flow therebetween so as to move a drug. A delivery rate of the drug and a current amount are correlated with each other. Therefore, a constant current device designed so as to cause the current to maintain a predetermined value regardless of an individual difference in impedance is generally used to apply a voltage between the electrodes. In general, if the density of the current is 0.2 mA/cm² or smaller, it is considered that the current is safe since biological influence such as irritation or the like is less likely to occur. Accordingly, the drug is administered by using the current amount of drug sticking area x 0.2 mA/cm² or smaller.

On the other hand, to maintain a constant current amount, it is necessary to apply a voltage corresponding to skin impedance between the electrodes. The skin impedance varies in a range from 1 kΩ to approximately 100 kΩ due to hydration, a change in an energizing time, a great individual difference, or a great site difference. If the skin impedance is 100 kΩ, a voltage of 20 V is needed to cause a current of 0.2 mA to flow between the electrodes. In general, it is understood that a voltage of several tens of volts or greater affects a human body. Thus, in many cases, a voltage exceeding 50 V is regarded as a dangerous voltage under normal environmental conditions. For this reason, to prevent pains or incidents in the iontophoresis device, a voltage, which can be applied between the electrodes is also limited to approximately 5 V or smaller, for example. Therefore, in a case of a patient with high skin impedance, especially an elderly person or the like who has dry skin, there is a problem in that a predetermined current is not caused to flow at a low voltage, that is, a prescribed amount of drug cannot be delivered.

The present invention is made in view of the above-described circumstances, and an object thereof is to enable the other drug administration device to compensate for an insufficient administration rate in one drug administration device for percutaneously administering a drug.

### Solution to Problem

To achieve the above-described object, a drug administration device according to an aspect of the present invention includes the following configurations. That is, there is provided a drug administration device which applies a voltage between electrodes so as to percutaneously administer a drug. The drug administration device includes
setting means for setting an administration rate of the drug,
determination means for determining a sharing administration rate so that the drug administration device and the other drug administration device share and realize the administration rate set by the setting means,
transmitting means for transmitting the administration rate which is determined by the determination means and which is shared with the other drug administration device, to the other drug administration device,
receiving means for receiving the administration rate of administration brought into practice from the other drug administration device, and
changing means for changing the administration rate of the other device so as to compensate for an insufficient administration rate, when one device between the drug administration device and the other drug administration device can no longer maintain the sharing administration rate.

### Advantageous Effects of Invention

According to the present invention, when a drug is percutaneously administered, another drug administration device can compensate for an insufficient administration rate used by one drug administration device. Therefore, the drug can be administered by using a desired administration rate without imposing a burden on a patient.

Other features and advantages of the present invention will become apparent from the following description with reference to the accompanying drawings.

### Brief Description of Drawings

The accompanying drawings are incorporated in the description, configure an aspect thereof, and illustrate embodiments of the present invention so as to describe principles of the present invention together with the description.
Fig. 1 is a general perspective view illustrating a percutaneous drug administration system according to a first embodiment.
Fig. 2a is a schematic bottom view of a patch.
Fig. 2b is a general perspective view when the patch is viewed from above.
Fig. 3 is a general perspective view when a controller is viewed from below.
Fig. 4 is a schematic block diagram of the controller.
Figs. 5A and 5B are views illustrating an example where drug administration is performed.
Fig. 6A is a flowchart illustrating a drug administration process performed by a master side controller.
Fig. 6B is a flowchart illustrating a drug administration process performed by the master side controller.
Fig. 7A is a flowchart illustrating a drug administration process performed by the master side controller.
Fig. 7B is a flowchart illustrating a drug administration process performed by the master side controller.
Fig. 8 is a flowchart illustrating a drug administration process performed by the master side controller.
Fig. 9A is a flowchart illustrating a drug administration process performed by a slave side controller.
Fig. 9B is a flowchart illustrating a drug administration process performed by the slave side controller.
Figs. 10a and 10b are views illustrating a display example in an "independent mode" of the master side controller.
Figs. 11a and 11b are views illustrating a display example in a "cooperating mode" of the master side controller.
Fig. 12 is a view illustrating an example of a current profile indicating an administration schedule employed by multiple drug administration devices.
Fig. 13 is a flowchart illustrating a process for realizing drug administration using the current profile indicating the administration schedule employed by the multiple drug administration devices.

### Description of Embodiments

Hereinafter, preferred embodiments according to the present invention will be described with reference to the accompanying drawings.

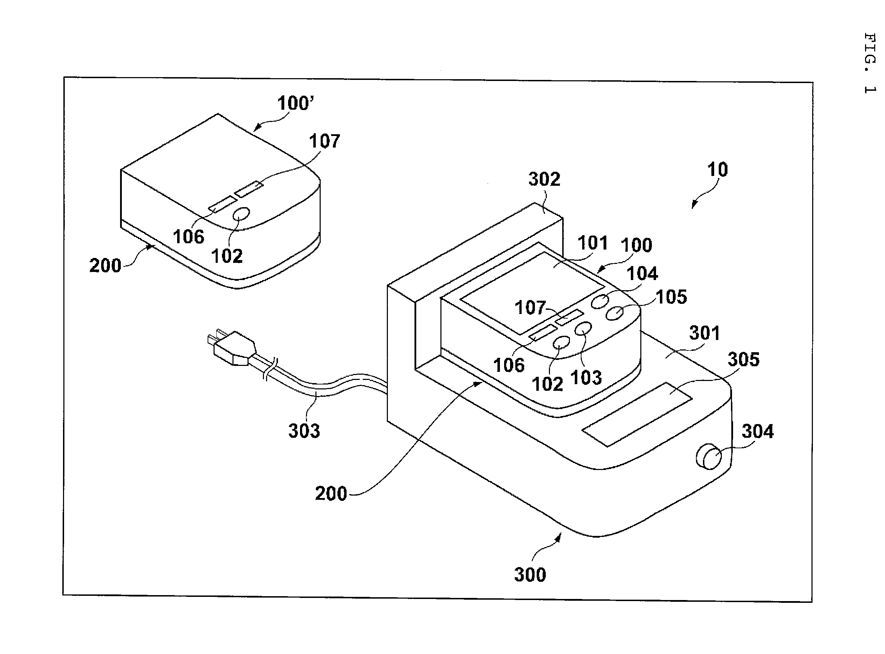

As illustrated in Fig. 1, a percutaneous drug administration system 10 according to a first embodiment is configured to include a patch 200, a controller 100, and a cradle 300 as a basic configuration. The controller 100 and the patch 200 are freely detachable, and are combined with each other (as will be described later) so as to configure a drug administration device. The controller 100 can communicate with a controller 100' configuring another drug administration device, and functions as a master side controller. A display 101 (to be described later) or various switches are not disposed in the controller 100', which functions as a slave (slave side controller) of the controller 100. The controller 100' and the patch 200 are attachable to and detachable from each other, and are assembled similarly to the controller 100 so as to configure another drug administration device which can communicate with the drug administration device configured to include the controller 100.

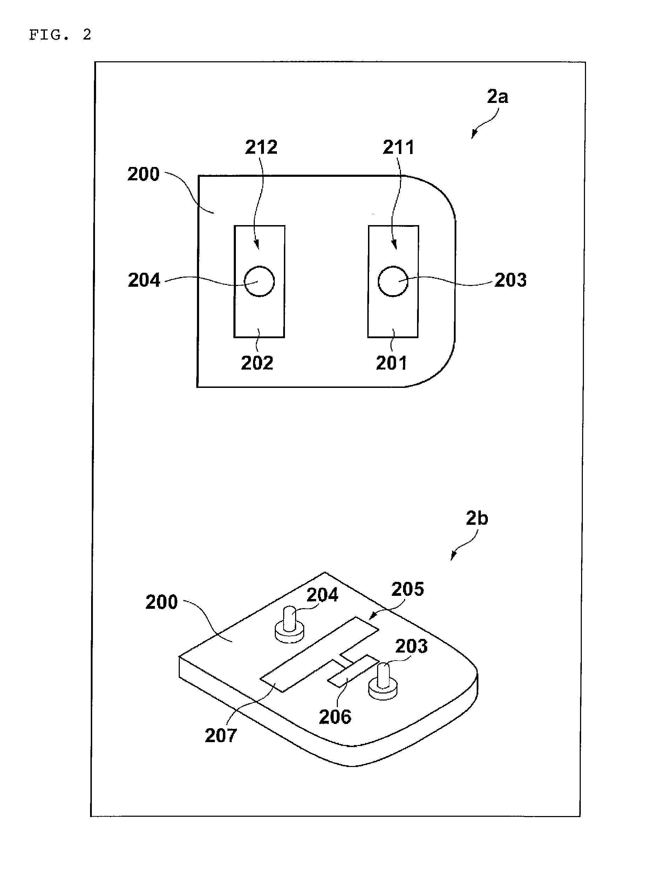

According to the percutaneous drug administration system 10 of the present embodiment, two drug administration devices, such as, a drug administration device having the patch 200 and the controller 100, integrated together, and another drug administration device having the patch 200 and the controller 100', integrated together, are caused to stick to a patient's skin. Then, a current is supplied to each of the integrated patches 200 from the controllers 100 and 100', thereby realizing iontophoresis for percutaneously administering a drug 211 (refer to Fig. 2) disposed in the patches 200. In this case, the controller 100 and the controller 100' communicate with each other, and perform a cooperating operation (as will be described later) to realize the set administration rate.

The iontophoresis is a method of using electrical energy so as to mainly promote an ionic drug to permeate a biological membrane, and is used in order to promote percutaneous absorption of drugs. The patch 200 has two reservoirs including electrodes (as will be described later), and is sealed with a drug, for example, in such a way that an anionic drug is contained in a cathode vessel and a cationic drug is contained in an anode vessel. The patch 200 sticks to the skin and a voltage with several volts is applied thereto so that a current with several hundred µA flows between the electrodes. In this manner, the drug is transferred to the skin, and concurrently, an endogenous ion making a pair with the drug is extracted into one reservoir from the skin. The other reservoir also induces ion exchange so as to establish an electrical circuit. Furthermore, if the skin is loaded with the voltage, water moves from an anode toward a cathode. According to this movement, the drug which does not dissociate therefrom is also percutaneously delivered.

As illustrated in Figs. 2a and 2b, the patch 200 has a donor reservoir 201, a return reservoir 202, a pair of connecting pins 203 and 204, and an RF tag chip 205. As illustrated in Fig. 2b of , the patch 200 has a thin plate shape, and the pair of connecting pins 203 and 204 are disposed to protrude upward on the upper surface of the patch 200. Here, in order to facilitate understanding of the description, a vertical direction in the percutaneous drug administration system 10 indicates a direction perpendicular to a contact surface between the patch 200 and the cradle 300 which are illustrated in Fig. 1, the controller 100 side indicates an upward direction, and the cradle 300 side indicates a downward direction. However, orientations used in practice are not limited thereto. In addition, both corners at one short side of a rectangular shape in a plan view are chamfered so as to have a round shape. This prevents a user from mistaking an orientation thereof when connecting the patch 200 to the controller 100.

The RF tag chip 205 is installed at a substantially central portion on an upper surface of the patch 200. The RF tag chip 205 is a passive RF tag which does not require an external power source such as a battery or the like, and has an IC chip 206 and an antenna 207. The antenna 207 is formed on the upper surface of the patch 200 by etching aluminum, copper, or silver or by using a printing method, and has a function of receiving a signal from the controller 100 and reflecting the signal. The IC chip 206 and the antenna 207 are covered with a film or the like (not illustrated).

The IC chip 206 is configured to include a high-frequency circuit operated at 800 MHz to 2. 54 GHz, a power recovery circuit, a memory control circuit, a memory, and the like (all are not illustrated). A power source of the RF tag chip 205 which is a passive RF tag reproduces DC power by causing the antenna 207 to receive a signal from outside by using the power recovery circuit and converting the signal into power. Since this passive RF tag is used, a power source such as a battery or the like is not required, thereby enabling the patch 200 to be miniaturized. In addition, since no power source is provided, an operable period of the RF tag chip 205 is no longer limited, thereby facilitating management of the patch 200. Without being limited to the passive RF tag, the RF tag chip 205 may employ an active RF tag, for example. For example, identification information (drug name) indicating types of contained drug and a parameter indicating a relationship between an administration amount of the contained drug and a current value (hereinafter, referred to as an administration rate parameter) are recorded on the IC chip 206.

The donor reservoir 201 and the return reservoir 202 are disposed at positions symmetrical with each other from substantially the center on a lower surface (surface in contact with a patient's skin) of the patch 200. The donor reservoir 201 is filled with the drug 211, and has a function as a positive electrode plate. That is, in the present embodiment, the donor reservoir 201 is an anode vessel, and the drug 211 is a drug having a positive ion such as a cationic drug or the like. The return reservoir 202 has the same structure as the donor reservoir 201, but has a function as a negative electrode plate, and forms a cathode vessel. The return reservoir 202 is filled with a physiological salt solution 212 instead of the drug 211. The physiological salt solution 212 has a negative ion. In a case of the patch 200 which administers a drug having the negative ion such as an anionic drug or the like, the cathode vessel serves as the donor reservoir.

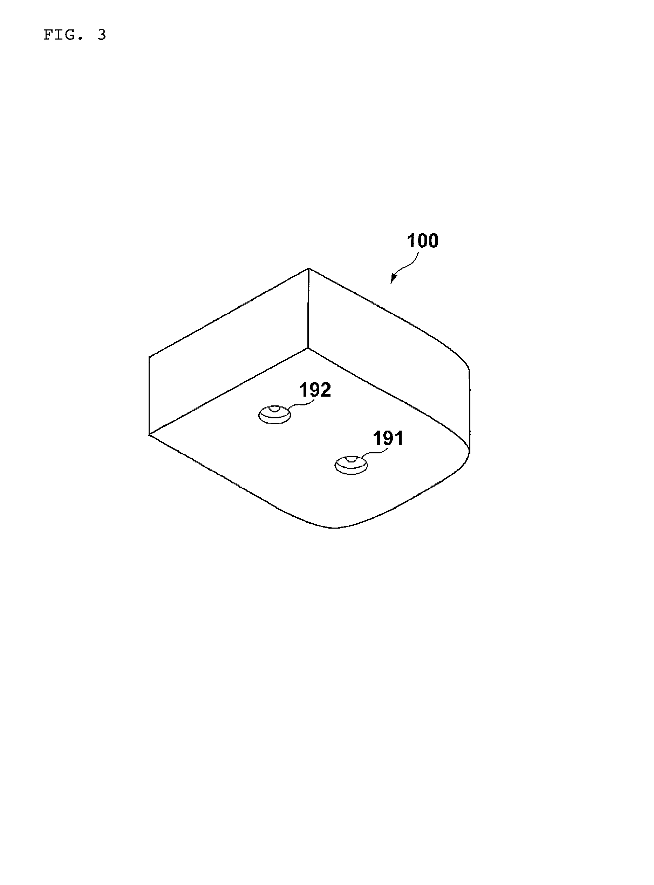

As described above, the connecting pins 203 and 204 are disposed to protrude on the upper surface of the patch 200, and are connected to the donor reservoir 201 and the return reservoir 202 inside the patch 200. In addition, the connecting pins 203 and 204 are inserted into connecting hooks 191 and 192 (refer to Fig. 3) having a concave shape in the controller 100, thereby providing a function of supplying electricity from the controller 100 to the donor reservoir 201 and the return reservoir 202. In addition, the pair of connecting pins 203 and 204 and the pair of connecting hooks 191 and 192 may be disposed at asymmetrical positions from the center on each installation surface. Alternatively, a diameter of the connecting pins 203 and 204 may be different from a diameter of the connecting hooks 191 and 192. This prevents a user from mistaking an orientation thereof when connecting the patch 200 to the controller 100.

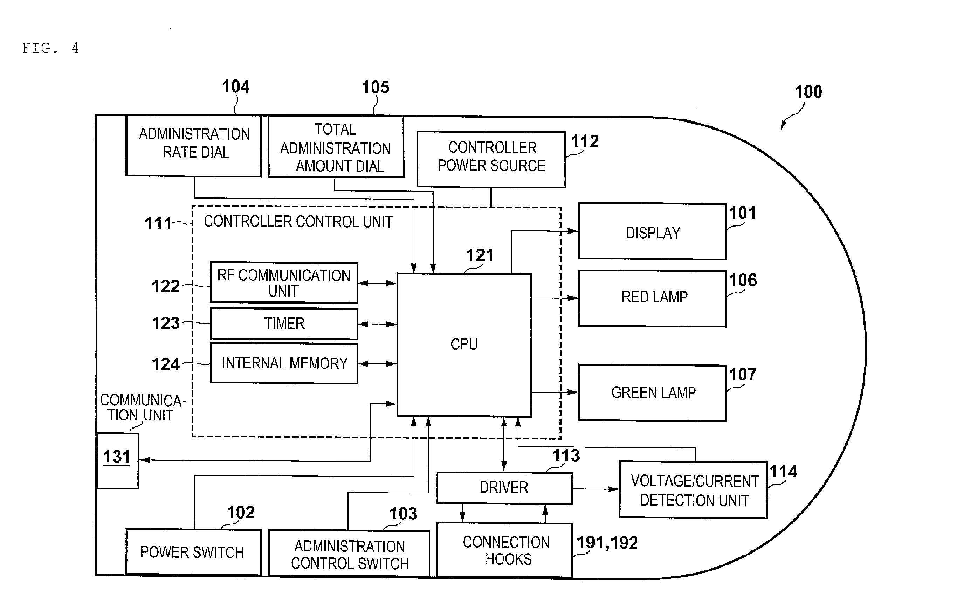

As illustrated in Figs. 1, 3, and 4, the controller 100 according to the present embodiment has the same shape as the patch 200 in a plan view. However, as a matter of course, the shape of the controller 100 or the like is not limited thereto. The controller 100 has the display 101 such as an LCD or the like, the pair of connecting hooks 191 and 192, a power switch 102, an administration control switch 103, a red lamp 106, a green lamp 107, an administration rate dial 104, and a total administration amount dial 105. In addition, the controller 100 internally includes a controller control unit 111 which is a control circuit of the controller 100, a controller power source 112, a driver 113, and a voltage/current detection unit 114.

As illustrated in Fig. 3, the connecting hooks 191 and 192 are holes which have a size and are arranged at positions on the lower surface in the controller 100 so as to enable engagement with the connecting pins 203 and 204. The connecting hooks 191 and 192 engage with the connecting pins 203 and 204, thereby providing a function of supplying power between the controller 100 and the patch 200. The connecting hook 191 is connected to the connecting pin 203, and the connecting hook 192 is connected to the connecting pin 204.

In a state where the power switch 102 is turned on, power is supplied from the controller power source 112 to the controller control unit 111, thereby enabling communication between the controller 100 and the patch 200 (RF tag chip 205), administration of the drug 211, or the like. In addition, in a state where the power switch 102 is turned off, the power is no longer supplied from the controller power source 112 to the controller control unit 111. As a type of the power switch 102, various types such as an alternate operation type, a momentary operation type, and the like are conceivable, but the type is not limited to both of these. Respective switches to be described below are not similarly limited thereto.

If the administration control switch 103 is in a turned-on state, the controller control unit 111 administers the drug 211 into a body of a patient. In a state where the administration control switch 103 is turned off, the controller control unit 111 stops and completes administration of the drug 211. A configuration may be adopted in which if the drug having an administration amount set by the total administration amount dial 105 is administered after the administration control switch 103 is turned on, the controller control unit 111 judges that the administration is completed, completes the administration of the drug 211, and turns off the administration control switch 103.

The red lamp 106 and the green lamp 107 are display lamps using a light emitting diode (LED), for example. The red lamp 106 has a function of notifying a patient or a qualified user such as a doctor, a nurse, and the like of a malfunction of the controller 100 by means of fast blinking. In addition, the red lamp 106 has a function of notifying the patient or the user of an insufficient residual power amount of the controller power source 112 by means of slow blinking. The green lamp 107 has a function of notifying the patient or the user of normal drug administration by means of blinking. However, uses of the red lamp 106 and the green lamp 107 are not limited thereto. In addition, a configuration may be adopted in which the notification function of the red lamp 106 or the green lamp 107 is realized by the display 101, and the red lamp 106 or the green lamp 107 is omitted.

The controller power source 112 is a secondary battery having a charging function, and may have a form and a size which allow the controller 100 to be portable. As the controller power source 112, a power source having a power storage function such as a dry cell, a button-type battery, and the like may be employed. In this case, the cradle 300 for charging is no longer required.

The controller control unit 111 has a central processing unit (CPU) 121 serving as a main control unit, an RF communication unit 122, a timer 123, and an internal memory 124. The driver 113, the voltage/current detection unit 114, and the like are connected to the controller control unit 111. The above-described respective functional units are realized by the CPU 121 reading a program and executing software processing in cooperation with the RF communication unit 122, the internal memory 124, the driver 113, and the like. The controller control unit 111 performs administration of the drug 211, at an administration rate and during an administration time, all of which are set by the administration rate dial 104 and the total administration amount dial 105. In addition, even if an administration rate is changed due to increase in resistance inside a body of a patient, variations in a power source voltage, and the like when the drug 211 is administered, the controller control unit 111 performs proportional integral differential (PID) control, proportional integral (PI) control, or the like for example, so as to maintain a set administration state.

The RF communication unit 122 communicates with the RF tag chip 205 of the patch 200. In the present embodiment, the RF communication unit 122 has a function of acquiring a type (drug name) or an administration rate parameter of the drug 211 from the patch 200 and transmitting the type or the parameter to the CPU 121. The internal memory 124 includes a nonvolatile memory such as a flash memory or the like, and the drug name, the administration rate parameters or the like received from the patch 200 via the RF communication unit 122 and the CPU 121 are written in the internal memory 124.

The driver 113 has a function of supplying a current to the connecting hooks 191 and 192, based on control information provided by the controller control unit 111. For example, the driver 113 can convert a supply current freely in time by using a pulse width modulation (PWM) method, and there is no limitation to a circuit on a current supply pattern. The voltage/current detection unit 114 detects a voltage and a current which are applied via the connecting hooks 191 and 192, and provides the CPU 121 with the detection result. This enables the CPU 121 to measure a current value and a voltage value between electrodes. The RF communication unit 122, the internal memory 124, the driver 113, and the voltage/current detection unit 114 may be integrated with the CPU 121. A communication unit 131 performs communication by establishing communication with another controller. The communication unit 131 may use wireless communication or wired communication. The present embodiment employs the wireless communication (for example, Bluetooth (Registered Trademark) or the like).

As illustrated in Fig. 1, the cradle 300 has a shape including a cradle main body 301 and a cradle wall portion 302 which have substantially rectangular parallelepiped shapes. Both corners at a side facing the cradle wall portion 302 having a rectangular shape in a plan view are chamfered so as to have a round shape. This shape is the same as that of the patch 200 and the controller 100, all of which are described above. A user can use this shape as a direction guide when the user integrates the patch 200 and the controller 100 with each other so as to set both of these on the cradle main body 301 as illustrated in Fig. 1.

The controller 100 described above is operated as the master side controller in the present embodiment. On the other hand, the controller 100' operated as the slave side controller is operated by receiving instructions, such as, an administration rate, administration start, administration completion, or the like from the controller 100. Therefore, as illustrated in Fig. 1, a configuration of the controller 100' is adopted in which the display 101, the administration control switch 103, the administration rate dial 104, and the total administration amount dial 105 are omitted from the configuration of the master side controller 100.

In addition, the cradle 300 includes a power code 303, a power switch 304, and an operation display unit 305. Drive power is supplied from a wall power source to the cradle 300 by the power code 303 extending from a rear surface. For example, an alternating current (AC) of 100 V or 200 V is used as a power source. Here, an example has been described in which a commercially available general wall power source is used, but the cradle 300 maybe driven by a battery. The power switch 304 is disposed on a side surface of the cradle main body 301, and supplies the power in a turned-on state. The power charges the controller power source 112 of the controller 100 (100') mounted on the cradle 300 as illustrated in Fig. 1. As a charging method, non-contact charging without using a contact may be performed, or charging may be performed by using electrodes. The operation display unit 305 notifies a user of charging in progress, charging completed, or the like.

The percutaneous drug administration system 10 according to the present embodiment is basically configured as described above. Next, a case will be described where the drug 211 is administered to a patient by using the percutaneous drug administration system 10.

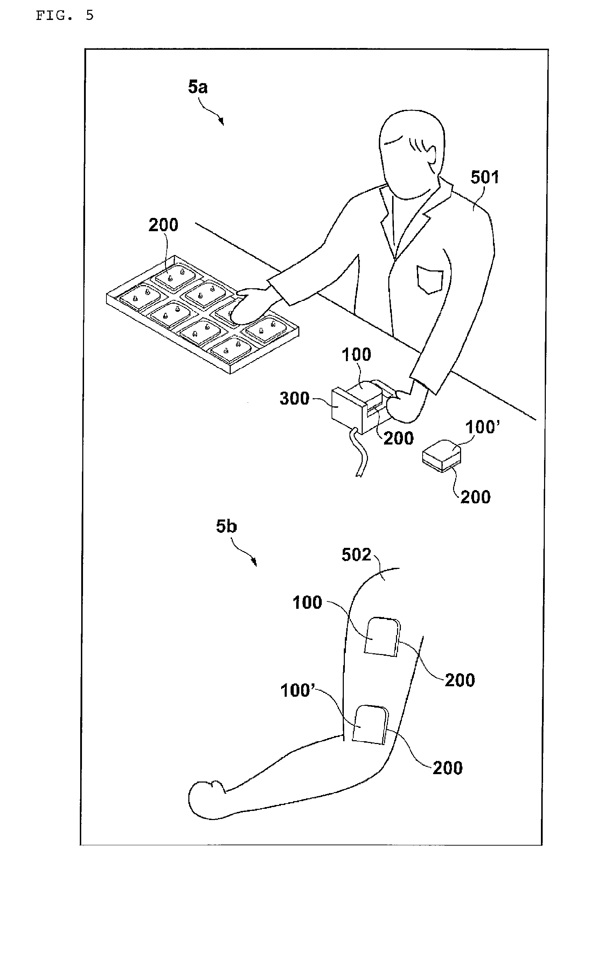

As illustrated by Fig. 5a, a user 501 first prepares the patch 200, the controller 100, the controller 100', and the cradle 300 in his or her hand. At a manufacturing stage, the donor reservoir 201 of the patch 200 is filled with the drug 211 in advance, and the return reservoir 202 is filled with the physiological salt solution 212 in advance. The above-described identification information, the parameter indicating the relationship between the administration rate and the current value, or the like is recorded in advance on the IC chip 206 of the patch 200 at the manufacturing stage.

As illustrated in Fig. 5a, the user 501 selects the suitable patch 200 from multiple patches, and integrates the patch 200 and the controller 100 with each other by causing the pair of connecting pins 203 and 204 to engage with the pair of connecting hooks 191 and 192. Similarly, the patch 200 containing the same drug as the above-described patch and the controller 100' are integrated with each other by causing the pair of connecting pins 203 and 204 to engage with the connecting hooks 191 and 192. The user 501 or a patient 502 detaches a predetermined protection film from the patch 200, and sticks the patch 200 and the controller 100 on the patient 502 (refer to Fig. 5b). The lower surface of the patch 200, that is, the surface in contact with the patient 502 has a moderate adhesive strength. Accordingly, the patch 200 and the controller 100 do not drop off from the patient 502. It is preferable to place the two drug administration devices at positions separated as far as possible. For example, it is preferable to respectively place the two drug administration devices on right and left arms, respectively.

The user 501 or the patient 502 turns on the administration control switch 103 of the controller 100. This action causes the CPU 121 of the controller 100 to read the parameter indicating a relationship between the administration amount and the current amount from the internal memory 124, to determine an amount of the current to be applied so as to satisfy the set administration speed, and to supply power from the driver 113 to the connecting hooks 191 and 192. In this manner, on the principle of electrophoresis, a current is generated which crosses the keratinous layer of the skin of the patient 502 from the donor reservoir 201 serving as a positive electrode, which then passes through the internal body side from the keratinous layer, and which flows to the return reservoir 202 serving as a negative electrode. Accordingly, the drug 211 which is positively charged moves similarly to the above-described current, but is absorbed into the body of the patient 502 through a site of the keratinous layer. Contrary to the above-described current, the negative ion contained in the physiological salt solution 212, for example, such as a chloride ion, crosses the keratinous layer of the patient 502 from the return reservoir 202, then passes through the internal body side from the keratinous layer, and moves to the donor reservoir 201. In addition, the drug 211 is administered to the patient 502 at the set administration rate and during the set administration time. In addition, the controller 100' practices the drug administration by determining an application current amount in accordance with the instruction received from the controller 100 via the communication unit 131 (as will be described later).

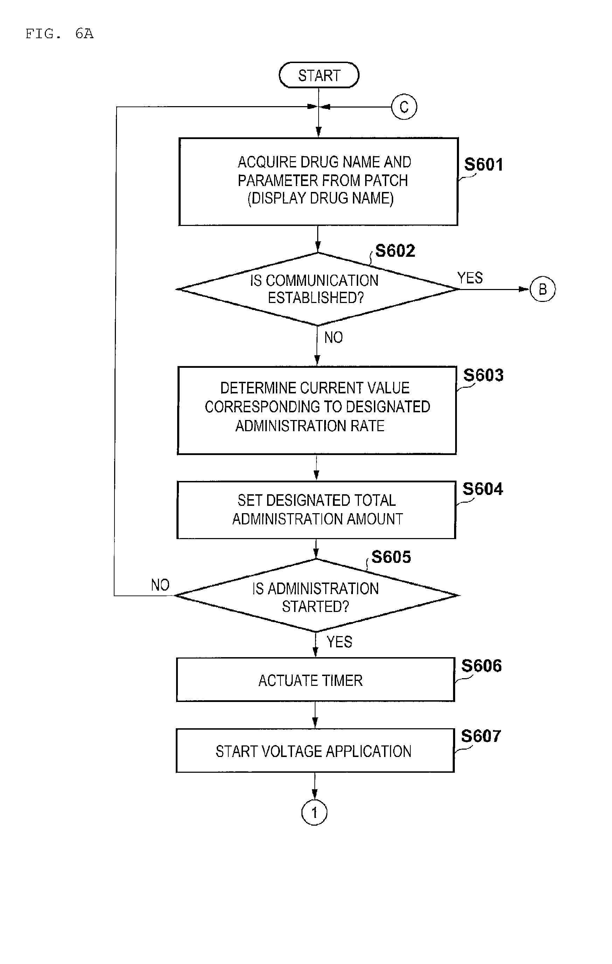

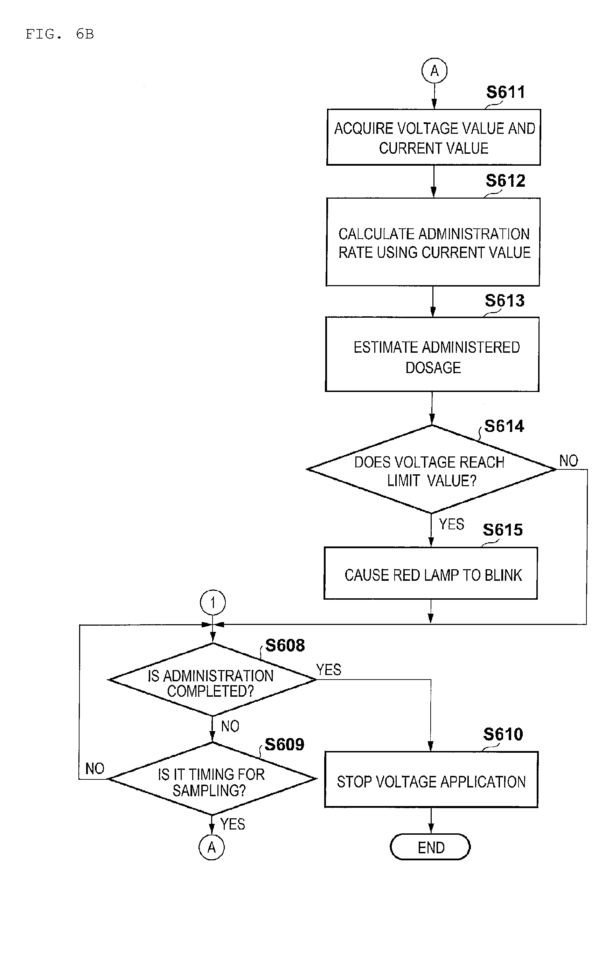

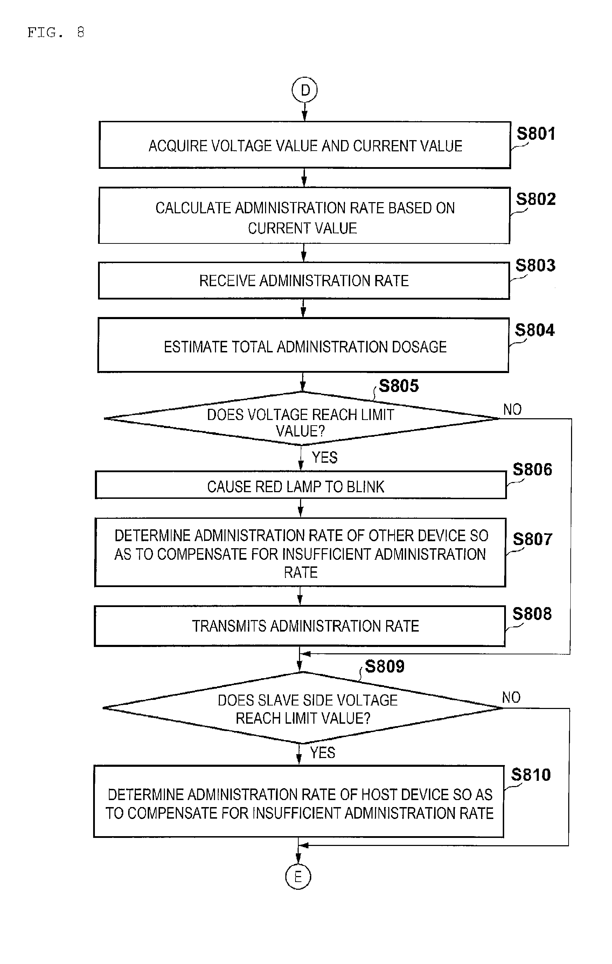

Hereinafter, referring to flowcharts in Figs. 6A to 9B, an operation of the drug administration system 10 according to the present embodiment will be described. First, referring to Figs. 6A to 8, an operation of the controller 100 serving as the master side controller will be described. The following process is realized in such a way that the power switch 102 of the controller 100 is turned on, power is supplied to the controller control unit 111, and the CPU 121 executes a predetermined program stored in the internal memory 124.

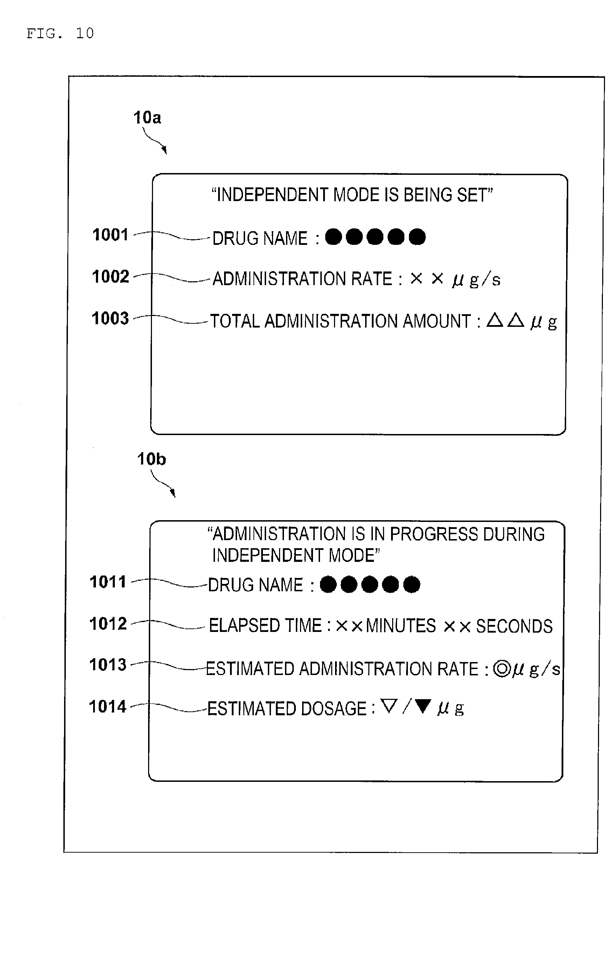

The CPU 121 first acquires a parameter indicating relationships between a type of drug (drug name), an administration rate, and a current value (for example, administration rate per 1 µA: x(µg/s)/µA, hereinafter, referred to as a rate parameter) from the RF tag chip 205 of the patch 200 via the RF communication unit 122 (Step S601). As illustrated in Fig. 10a, for example, the drug name acquired here is displayed on the display 101 as a drug name display 1001. The user 501 or the patient 502 (hereinafter, simply referred to as a user) can identify the drug administered by the patch 200 placed on the controller 100.

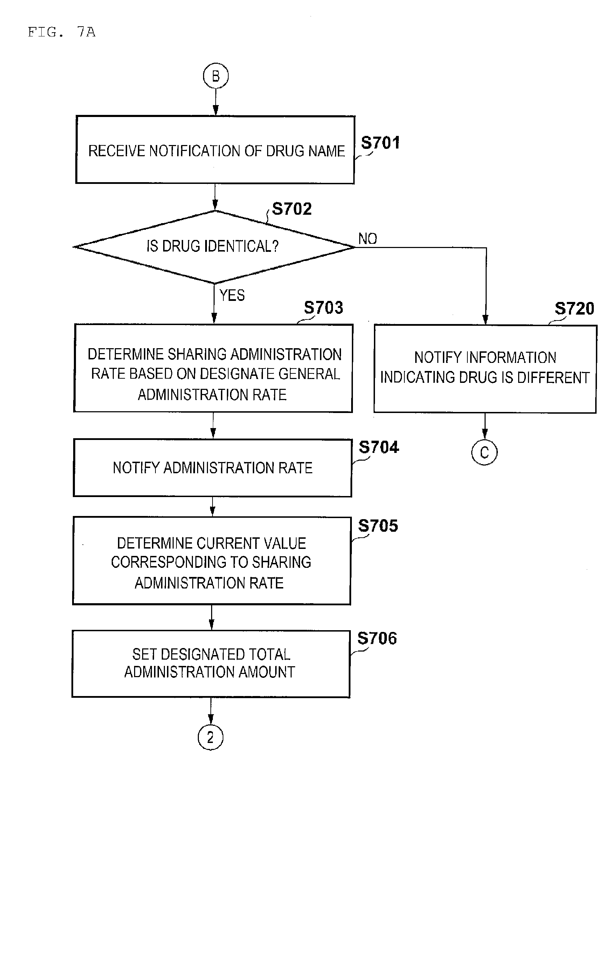

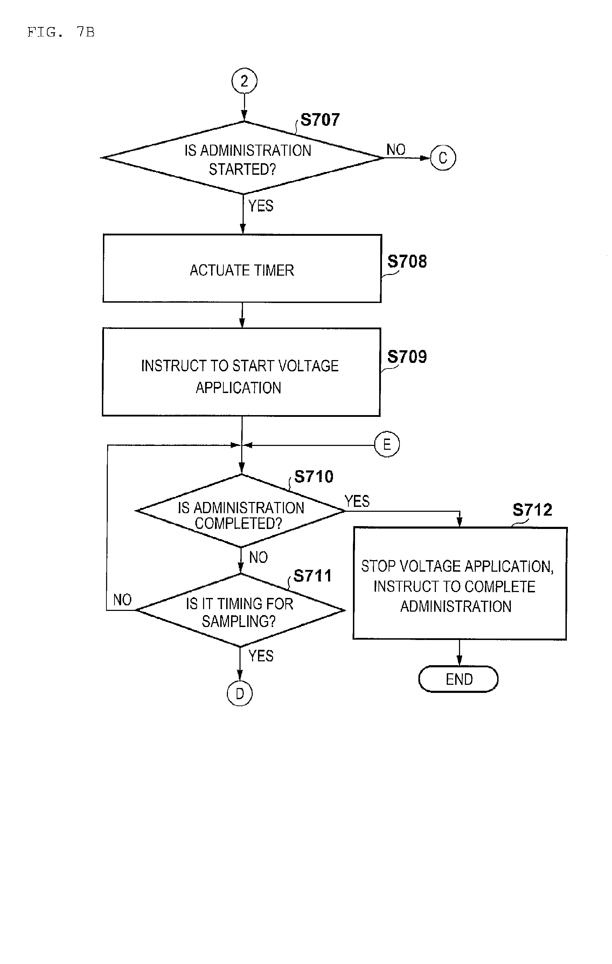

Next, the CPU 121 judges whether communication with the slave side controller 100' is established by the communication unit 131 (Step S602). If the communication is established, the process proceeds to Step S701 (refer to Fig. 7A). The controller 100 may have a switch which switches between a "cooperating mode" for practicing administration in cooperation with another drug administration device and an "independent mode" in which the controller 100 independently practices drug administration. In this manner, during the "cooperating mode" and when the communication is established, the process may proceed to Step S701.

Steps S603 to S615 represent an operation performed by the controller 100 during the independent mode, and a display example using the display 101 in this case is illustrated in Figs. 10a and 10b. The CPU 121 detects a setting state of the administration rate dial 104, and displays the administration rate instructed by the administration rate dial 104 as an administration rate display 1002 (Step S603). If the user operates the administration rate dial 104, the administration rate display is updated accordingly. The administration rate corresponding to a current value varies depending on a type of drug. Thus, the CPU 121 calculates a value of the current to be supplied between the electrodes for realizing the designated administration rate, by using the administration rate parameter acquired from the patch 200 in Step S601.

Then, the CPU 121 detects a setting state set by the total administration amount dial 105, and acquires a total administration amount instructed by an operator (Step S604). The acquired total administration amount is displayed as a total administration amount display 1003. Thereafter, the CPU 121 waits for the administration control switch 103 to be turned on (Step S605). While the administration control switch 103 is turned off, the processes are repeated from Step S601 to Step S605. If the administration control switch 103 is turned on, the process proceeds from Step S605 to Step S606 so as to practice the drug administration. As the administration rate and the total administration amount, a value set at the time when the process proceeds to Step S606 is adopted.

While the processes are repeated from Step S601 to Step S605, the CPU 121 causes the green lamp 107 to blink so as to show that the administration rate or the total administration amount is being set. Then, if the administration control switch 103 is turned on and the process proceeds to Step S606 and the subsequent steps, the CPU 121 causes the green lamp 107 to be continuously lit so as to inform that the drug is being administered. In addition, while the administration rate or the total administration amount is set, as illustrated in Fig. 10a, information indicating that the "individual mode is being set" is displayed. If the drug is being administered, as illustrated in Fig. 10b, information indicating that the "administration is in progress in the independent mode" is displayed.

If the administration control switch 103 is turned on, the CPU 121 starts to administer the drug held in the patch 200. The CPU 121 first actuates the timer 123, and measures a time elapsed from the start of administration (Step S606). Then, the CPU 121 instructs the driver 113 so as to supply a current value corresponding to the administration rate set in Step S603. The driver 113 includes a constant current source for supplying a current according to the instructed current value, and applies a required voltage to the connecting hooks 191 and 192. In addition, at this timing, the CPU 121 causes the display 101 to switch display content on a display screen used during the administration as illustrated in Fig. 10b, and causes the display 101 to display the drug name acquired at the time when the process proceeds to Step S606 as a drug name display 1011.

The CPU 121 judges whether or not the drug administration is completed (Step S608). For example, whether the drug administration is completed can be judged by judging whether an administration amount estimated through an estimation process (to be described later) of the drug (Step S613) reaches a total administration amount designated in Step S604. Alternatively, a required administration time may be calculated by dividing the total administration amount set in Step S604 by the administration rate set in Step S603. In this manner, when a time set based on this calculation elapses, it may be judged that the administration is completed. If it is judged that the drug administration is completed, the process proceeds to Step S610, and this process is completed after stopping current application for administering the drug.

On the other hand, if the administration is not completed, the process proceeds to Step S609, and it is judged whether it is the time for sampling. If it is not the time for sampling, the process returns to Step S608. On the other hand, if it is the time for sampling, the process proceeds to Step S611. The time for sampling is set to occur at a predetermined time interval, for example, every second.

If it is judged as the time for sampling, the CPU 121 acquires a voltage value and a current value between the connecting hooks 191 and 192, that is, between electrodes, from the voltage/current detection unit 114 (Step S611). Then, the CPU 121 calculates an estimated administration rate by using the parameter acquired in Step S601 and the current value acquired in Step S611 (Step S612). Furthermore, the CPU 121 estimates an administered dosage at the present time, based on an administration rate from the start of administration to the present time (Step S613).

The CPU 121 causes the display 101 to display an administration rate (estimated administration rate) calculated in Step S612, as an estimated administration rate display 1013. In addition, the CPU 121 causes the display 101 to display the administered dosage which is estimated in Step S613, as an estimated dosage display 1014. In addition, the CPU 121 acquires the elapsed time from the start of administration from the timer 123, and causes the display 101 to display the elapsed time as an elapsed time display 1012.

Next, the CPU 121 judges whether or not the current value detected in Step S611 reaches the maximum limit value, and if the current value reaches the maximum limit value, the CPU 121 draws a user's attention by causing the red lamp 106 to blink (Steps S614 and S615).

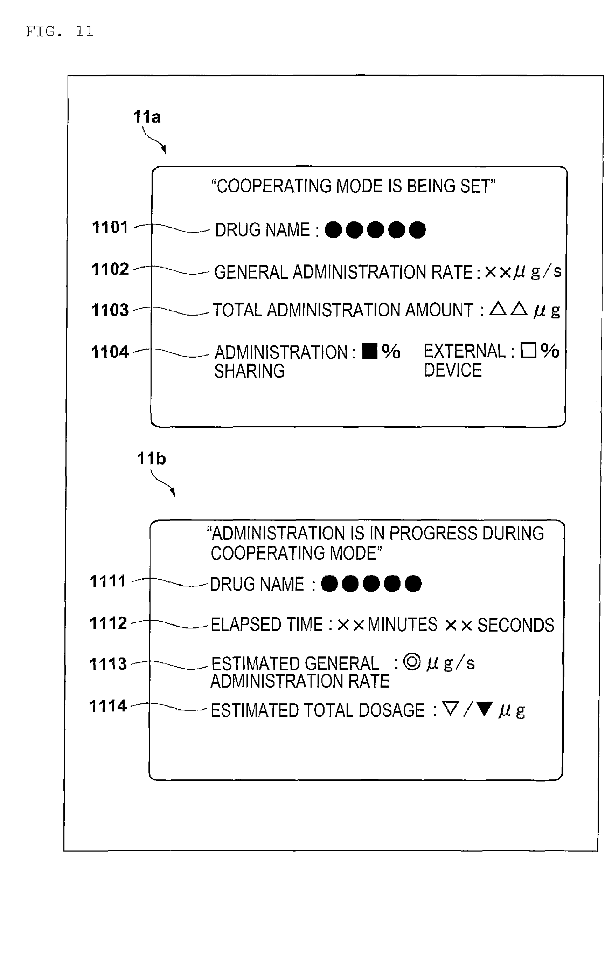

The processes illustrated in Figs. 7A to 8 represent operations of the controller 100 during the cooperating mode, and a display example using the display 101 in this case is illustrated in Figs. 11a and 11b. The display of the drug name (drug name display 1101) is a display of the drug name acquired in Step S601. If it is confirmed that the communication is established in Step S602, the CPU 121 acquires the drug name of the patch mounted on the slave side drug administration device from the slave side controller (Step S701). The CPU 121 judges whether or not drugs contained in the patches mounted on the controller 100 and the controller 100' are coincident with each other (Step S702). When not coincident, the CPU 121 causes the display 101 to display the result, and the process returns to Step S601 (Step S720).

If the drugs are coincident with each other, the CPU 121 detects the setting state of the administration rate dial 104, and the CPU 121 acquires the administration rate instructed by the administration rate dial 104 as a general administration rate. Then, the sharing administration rate between the master side and the slave side is determined in accordance with a sharing ratio of the administration rates employed by the master side controller and the slave side controller (Step S703). In embodiments, each side shares 50%. However, the sharing ratio is not limited thereto. In addition, a configuration may be adopted in which a user can designate the sharing ratio. If the sharing ratio is 50% and when the general administration rate is set to 2 µg/s, the respective controllers take charge of 1 µg/s. The CPU 121 causes the display 101 to display the sharing ratio as an administration sharing display 1104.

Then, the CPU 121 notifies the controller 100' of a slave side administration rate determined in Step S704 (Step S704). If the general administration rate is 2 µg/s and the sharing ratio is 50% as described above, the CPU 121 notifies the slave side controller 100' that the administration rate is 1 µg/s. In addition, if a user operates the administration rate dial 104, the general administration rate is updated. Accordingly, the slave side controller 100' is notified of an updated sharing administration rate, and a general administration rate display 1102 is updated. Next, the CPU 121 determines a current value for realizing the sharing administration rate by using the sharing administration rate and the administration rate parameter acquired from the patch 200 in Step S601 (Step S705).

Next, the CPU 121 detects the setting state set by the total administration amount dial 105, and acquires the total administration amount instructed by an operator (Step S706). The acquired total administration amount is displayed as a total administration amount display 1103. Thereafter, the CPU 121 waits for the administration control switch 103 to be turned on (Step S707). While the administration control switch 103 is turned off, the processes from Step S601 are repeated. If the administration control switch 103 is turned on, the process proceeds from Step S707 to Step S708. The most current determined administration rate and total administration amount forms a value set at the time when the process proceeds to Step S708 and is adopted.

While the processes are repeated from Steps S601 and S602 to Step S707, the CPU 121 causes the green lamp 107 to blink so as to show that the administration rate or the total administration amount is being set. Then, if the administration control switch 103 is turned on and the process proceeds to Step S606 and the subsequent steps, the CPU 121 causes the green lamp 107 to be continuously lit so as to inform that the drug is being administered. In addition, while the administration rate or the total administration amount is being set, as illustrated in Fig. 11a, the "cooperating mode is being set" is displayed. If the drug is being administered, as illustrated in Fig. 11b, the "administration is in progress during the cooperating mode" is displayed.

If the administration control switch 103 is turned on, the CPU 121 starts to administer the drug held in the patch 200 (Step S707). The CPU 121 first actuates the timer 123, and measures a time elapsed from the start of administration (Step S708). Then, the CPU 121 instructs the driver 113 so as to supply a current value for realizing the sharing administration rate set in Step S705 (Step S709). The driver 113 includes a constant current source for supplying a current according to the instructed current value, and applies a required voltage to the connecting hooks 191 and 192. In addition, at this timing, the CPU 121 causes the display 101 to switch display content on an administration display screen as illustrated in Fig. 11b, and causes the display 101 to display the drug name acquired at the time when the process proceeds to Step S708, as a drug name display 1111.

The CPU 121 judges whether or not the drug administration is completed (Step S710). For example, whether the drug administration is completed can be judged by judging whether an administration amount estimated through an estimation process (to be described later) of the drug (Step S804) reaches a total administration amount set in Step S706. Alternatively, a required administration time may be calculated by dividing the total administration amount set in Step S706 by the general administration rate set in Step S704. In this manner, when a time set based on this calculation elapses, it may be judged that the administration is completed. If it is judged that the drug administration is completed, the process proceeds to Step S712, and the process is completed after stopping current application for administering the drug and instructing the slave side controller 100' to complete the administration.

On the other hand, if the administration is not completed, the process proceeds to Step S711, and it is judged whether or not it is the time for sampling. If it is not the time for sampling, the process returns to Step S710. On the other hand, if it is the time for sampling, the process proceeds to Step S801. The time for sampling is set to occur at a predetermined time interval, for example, every second.

If it is judged as the time for sampling, the CPU 121 acquires a voltage value and a current value between the connecting hooks 191 and 192, that is, between electrodes, from the voltage/current detection unit 114 (Step S801). Then, the CPU 121 calculates an estimated administration rate by using the parameter acquired in Step S601 and the current value acquired in Step S801 (Step S802). In addition, the CPU 121 receives the administration rate employed in the controller 100' from the slave side controller 100' (Step S803). Then, a general administration rate is calculated together with the administration rate calculated in Step S802. Based on the general administration rate up to the present time, the CPU 121 estimates an administered dosage at the present time (Step S804). The CPU 121 causes the display 101 to display the elapsed time acquired from the timer 123, the calculated general administration rate, and the estimated administered dosage, respectively, as an elapsed time display 1112, an estimated general administration rate display 1113, and an estimated total dosage display 1114 (refer to Fig. 11b).

Next, the CPU 121 judges whether the voltage value detected in Step S801 reaches the maximum limit value, and if the voltage value reaches the maximum limit value, the CPU 121 causes the red lamp 106 to blink so as to attract a user's attention (Steps S805 and S806). In addition, the CPU 121 instructs the slave side controller 100' to update the administration rate so as to compensate for an insufficient administration rate caused by the voltage reaching the limit value (Steps S807 and S808). For example, in a case of the above-described setting, if the administration rate is 0.8 µs and the voltage value reaches the upper limit, the CPU 121 instructs the slave side controller 100' to set the administration rate to be 1.2 µs.

In addition, the CPU 121 judges whether the notification that the voltage value has reached an upper limit value is received from the slave side controller 100' (Step S809). When the notification that the voltage value has reached the upper limit value is received from the slave side controller 100', if the administration rate received from the slave side in Step S803 is insufficient, the CPU 121 changes its own administration rate so as to compensate for the insufficient administration rate.

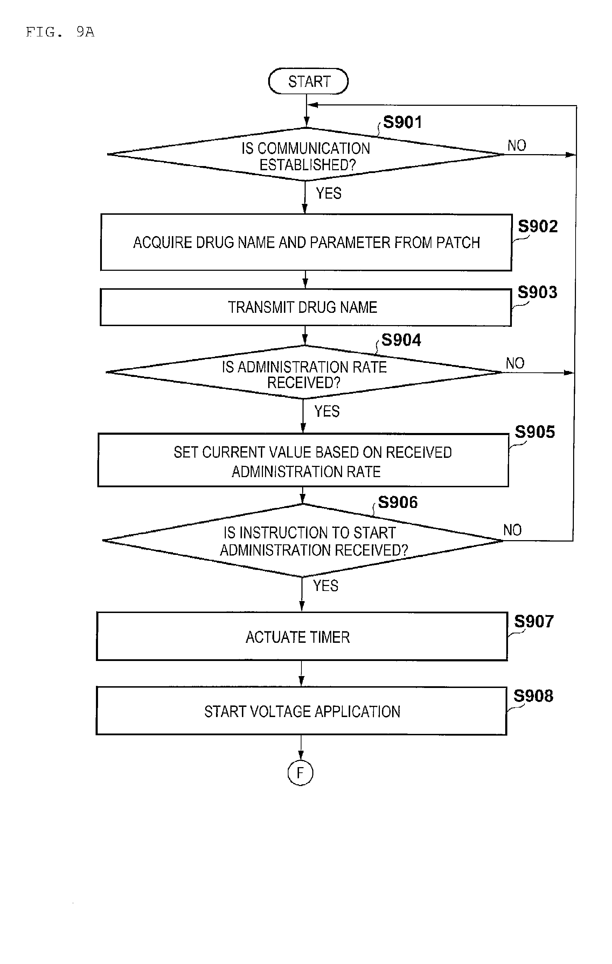

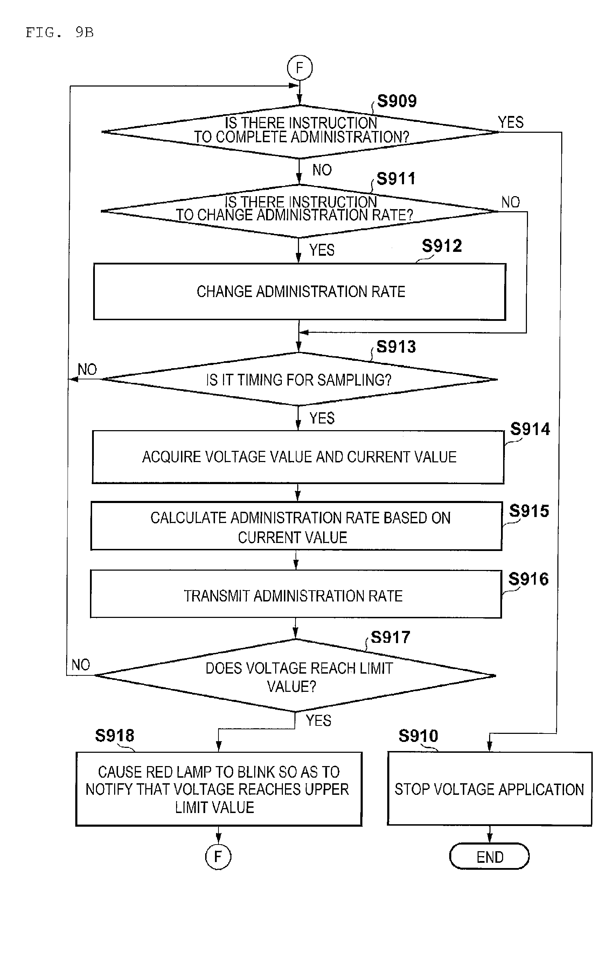

Next, an operation of the CPU 121 in the slave side controller 100' will be described with reference to flowcharts in Figs. 9A and 9B. The CPU 121 waits for communication with the master side controller 100 being established (Step S901). If the communication is established, the CPU 121 acquires the drug name and the rate parameter from the patch 200, and notifies the master side controller 100 of the drug name (Steps S902 and S903).

Next, the CPU 121 waits for the notification of the administration rate (Step S704) being received from the master side controller 100 via the established communication (Step S904). If the CPU 121 receives the administration rate, the CPU 121 calculates a current value corresponding to the received administration rate by using the received administration rate and the rate parameter acquired from the patch 200 in Step S902 (Steps S904 and S905). Thereafter, the CPU 121 waits for an instruction to start administration being received from the master side controller 100 via the communication (Step S906).

While the processes from Step S901 to Step S906 are repeated, the CPU 121 causes the green lamp 107 to blink so as to show that the administration rate is being set via the communication. Then, if the CPU 121 receives the instruction to start administration and the process proceeds to Step S907 and the subsequent Steps, the CPU 121 causes the green lamp 107 to light continuously so as to notify a user that the drug is being administered.

If the CPU 121 receives the instruction to start administration, the CPU 121 starts to administer the drug held in the patch 200. The CPU 121 first actuates the timer 123, and measures a time elapsed from the start of administration (Step S907). Then, the CPU 121 instructs the driver 113 so as to supply a current value corresponding to the administration rate set in Step S906 (Step S908). The driver 113 includes a constant current source for supplying a current according to the instructed current value, and applies a required voltage to the connecting hooks 191 and 192.

The CPU 121 judges whether an instruction to complete drug administration (Step S712) is received from the master side controller 100 (Step S909). If it is judged that the instruction to complete drug administration is received, the process proceeds to Step S910, and this process is completed after stopping current application for administering the drug. On the other hand, if the instruction to complete drug administration is not received, the process proceeds to Step S911, the CPU 121 judges whether an instruction to change the administration rate (Step S807) is received from the master side controller 100. If the instruction to change the administration rate is received, the CPU 121 calculates and sets a current value corresponding to the changed administration rate by using the rate parameter acquired in Step S902.

Next, the CPU 121 judges whether or not it is the time for sampling (Step S913). If it is not the time for sampling, the process returns to Step S910. On the other hand, if it is the time for sampling, the process proceeds to Step S614. The time for sampling is set to occur at a predetermined time interval, for example, every second.

If it is judged as the time for sampling, the CPU 121 acquires a voltage value and a current value between the connecting hooks 191 and 192, that is, between electrodes, from the voltage/current detection unit 114 (Step S914). Then, the CPU 121 calculates an estimated administration rate by using the parameter acquired in Step S902 and the current value acquired in Step S14 (Step S915), and transmits the estimated administration rate to the master side controller 100 (Step S916).

Next, the CPU 121 judges whether or not the voltage value detected in Step S914 reaches the maximum limit value, and if the voltage value reaches the maximum limit value, the CPU 121 causes the red lamp 106 to blink so as to attract a user's attention (Steps S917 and S918). In addition, at this time, the CPU 121 of the controller 100' transmits notification that the voltage between the electrodes has reached the upper limit value to the master side controller 100. The notification that the voltage has reached the upper limit value is used to maintain the general administration rate in Steps S809 and S810 described above.

According to the above-described control, the master side controller 100 and the slave side controller 100' are operated in cooperation with each other. In this manner, even when a patient has high skin resistance, the drug can be stably administered by using the administration rate as instructed.

Alternatively or in addition, if the master side shares 100%, the slave side practices drug administration when the master side can no longer maintain the administration rate of 100%.

A configuration has been described in which the administration rate or the total administration amount of drug is set manually. However, as disclosed in PTL 2, a configuration may be adopted in which a current profile or the like is acquired from the cradle 300 and the application voltage is controlled according to the current profile.

In addition, the controllers 100 and 100' acquire a parameter indicating a relationship between the administration rate and the current value by means of RF communication with the patch 200. However, configurations are not limited thereto. For example, a table, in which a "type of drug" and a "parameter indicating the relationship between the administration rate and the current value" are associated with each other, may be held in the internal memory 124 of the controller, and a parameter may be acquired with reference to the table. In this case, the type of drug (drug name) may be acquired by means of the RF communication with the patch 200, or, for example, an operator may manually designate the type of drug by disposing a drug designation dial. Furthermore, the controllers 100 and 100' may read a pin arrangement or the like of the patch 200, and may detect the type of drug.

Furthermore, the current profile may be set so as to administer a predetermined administration amount by alternately supplying power to the master side and at least one slave side controller at predetermined time intervals (in a case of three or more slave side controllers, in accordance with setting content). In this case, the master side controller 100 notifies the slave side controller of the instruction to start administration and the administration rate of a drug according to the current profile.

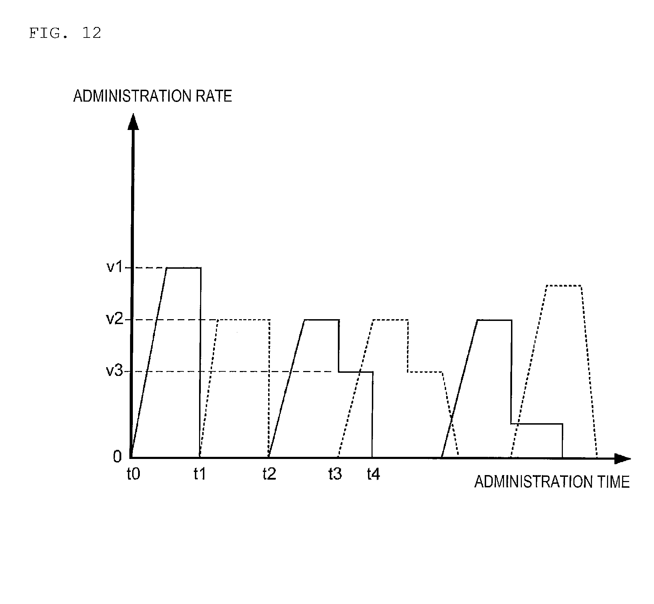

For example, it is assumed that the master side controller 100 receives a profile as illustrated in Fig. 12. This profile has a recorded pattern (illustrated by a solid line) of drug administration practiced by a master side device and a recorded pattern (illustrated by a dashed line) of drug administration practiced by a slave side device. If the drug administration starts at time t0, the master side controller 100 gradually applies a voltage so as to obtain a current output corresponding to an administration rate v1, and temporarily completes the voltage application at time t1. Then, at the time t1, the master side controller 100 instructs the slave side controller 100' to practice the administration by using an administration rate v2. In accordance with this instruction, the slave side controller 100' practices the administration by using the administration rate v2. At the time t2, the master side controller 100 instructs the slave side controller 100' to complete the administration, and the master side controller 100 starts the administration in succession by using the administration rate v2. Thereafter, at time t3, the master side controller 100 changes its own administration rate to v3, and instructs the slave side controller 100' to practice the administration by using the administration rate v2. In addition, at time t4, the master side controller 100 sets the current value between the electrodes to zero, and stops the administration.

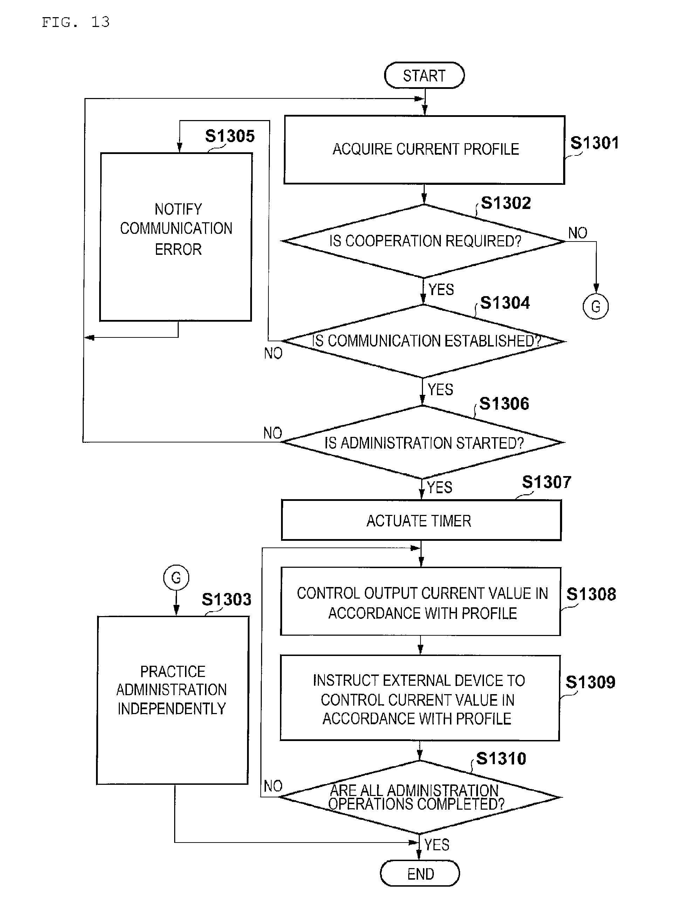

Fig. 13 is a flowchart illustrating processes performed by the master side controller 100 to realize the administration in accordance with a current profile having a recorded administration schedule employed by multiple drug administration devices as described above. The slave side controller 100' is adapted to apply a current in accordance with a drive command transmitted from the master side controller 100.

In Step S1301, the controller control unit 111 acquires the current profile, and holds the current profile in the internal memory 124. The current profile may be acquired from the cradle 300 by employing a configuration as disclosed in PTL 2, or a configuration may be adopted in which a computer (PC) can set the current profile via a predetermined communication interface (such as a USB or the like).

In Step S1302, the controller control unit 111 judges whether the current profile acquired in Step S1301 is a schedule for administration practiced by an independent drug administration device, or a schedule for administration which requires cooperation between multiple drug administration devices. If the administration is independently practiced, the process proceeds to Step S1303, and the controller control unit 111 controls the driver 113 to practice the administration according to the current profile.

On the other hand, if the current profile, as illustrated in Fig. 12, is the schedule for administration which requires cooperation, the process proceeds to Step S1304. In Step S1304, the controller control unit 111 establishes communication with the cooperating drug administration device (slave side controller 100'). If the communication is successfully established or has already been successfully established, the process proceeds from Step 1304 to Step 1306. On the other hand, if the communication is not established, the process proceeds to Step S1305. The red lamp 106 is, for example, caused to blink so as to notify a user of a communication error, and then the process returns to Step S1301. In Step S1306, it is judged whether an instruction to start administration is given (whether the administration control switch 103 is turned on), and, if the instruction to start administration is given, the process proceeds to Step S1307. If the instruction to start administration is not given, the process returns to Step S1301.

Steps S1307 and the subsequent steps represent processes in which cooperating administration is practiced according to the current profile. In Step S1307, the controller control unit 111 actuates the timer 123. In Step S1308, the controller control unit 111 drives the driver 113 in accordance with a timer value of the timer 123 and a profile allocated to its own device within the current profile, and controls an output current. In addition, in Step S1309, the controller control unit 111 instructs the other device to have a current value in accordance with the timer value of the timer 123 and a profile allocated to the other device within the current profile.

For example, in a case of the current profile illustrated in Fig. 12, the controller control unit 111 controls the driver 113 so that the administration rate reaches v1 with predetermined inclination at the timing t0 of the timer 123, and stops the administration at the timing t1. Then, at the timing t1, via the communication unit 131, the controller control unit 111 instructs the slave side controller 100' so that the administration rate reaches v2 with inclination indicated by the profile.

If all administration operations recorded in the current profile are completed through the above-described processes, this process is completed (Step S1310). Hitherto, a case of one slave side controller 100' has been described. However, two or more slave side controllers 100' may be provided. In this case, it is necessary to cause the current profile to distinguish a master side controller, a slave side controller A, and a slave side controller B from one another, and it is necessary to specify which slave side controller is to receive an instruction to control a current value in Step S1309. However, these processes are apparent to those skilled in the art.

The master side controller 100 performs the above-described operations according to the acquired current profile. In this manner, the master side controller 100 can easily perform processes according to a complicated administration schedule using multiple administration devices.

The present invention is not limited to the above-described embodiments, and can be changed and modified in various ways without departing from the spirit and the scope of the present invention. Therefore, the following claims are appended herein to define the scope of the present invention publically.

## Claims

1. A drug administration device which applies a voltage between electrodes so as to percutaneously administer a drug, the drug administration device comprising:
setting means for setting an administration rate of the drug;
determination means for determining a sharing administration rate so that the drug administration device and the other drug administration device share and realize the administration rate set by the setting means;
transmitting means for transmitting the administration rate which is determined by the determination means and which is shared with the other drug administration device, to the other drug administration device;
receiving means for receiving the administration rate of administration brought into practice from the other drug administration device; and
changing means for changing the administration rate of the other device so as to compensate for an insufficient administration rate, when one device between the drug administration device and the other drug administration device can no longer maintain the sharing administration rate.

2. The drug administration device according to Claim 1, further comprising:
checking means for checking the drug set in the drug administration device and the other drug administration device,
wherein when the checking means judges that a different drug is set, the checking means prohibits the shared practice.

3. The drug administration device according to Claim 1 or 2,
wherein when a voltage value between the electrodes reaches an upper limit value, the changing means changes the administration rate in the other drug administration device so as to compensate for the insufficient administration rate in the drug administration device.

4. The drug administration device according to any one of Claims 1 to 3,
wherein when the changing means receives information that the voltage value between the electrodes reaches the upper limit value from the other drug administration device, the changing means changes the administration rate in the drug administration device so as to compensate for the insufficient administration rate in the other drug administration device.

5. A drug administration system having first and second drug administration devices which apply a voltage between electrodes so as to percutaneously administer a drug, the drug administration system comprising:
setting means for setting an administration rate of the drug;
determination means for determining each sharing administration rate so that the first and second drug administration devices share and realize the administration rate set by the setting means;
practice means for causing the first and second drug administration devices to practice drug administration using the administration rate determined by the determination means; and
changing means for changing the administration rate of the other device so as to compensate for an insufficient administration rate, when one device between the first and second drug administration devices can no longer maintain the sharing administration rate determined by the determination means.

6. A drug administration device which applies a voltage between electrodes so as to percutaneously administer a drug, the drug administration device comprising:
acquisition means for acquiring a profile in which a drug administration pattern employed by multiple drug administration devices is recorded;
practice means for causing the drug administration device to practice administration in accordance with the administration pattern which indicates an administration rate and an administration time of the drug, which is recorded in the profile, and which is to be used for the practice of the drug administration device; and
instruction means for instructing the other drug administration device to follow the administration rate, to start the administration, and to stop the administration in accordance with the administration pattern which is recorded in the profile and which is employed by the other drug administration device.

7. A control method of a drug administration device which applies a voltage between electrodes so as to percutaneously administer a drug, the control method comprising:
a setting step of setting an administration rate of the drug;
a determination step of determining a sharing administration rate so that the drug administration device and the other drug administration device share and realize the administration rate set in the setting step;
a transmitting step of transmitting the administration rate which is determined in the determination step and which is shared with the other drug administration device, to the other drug administration device;
a receiving step of receiving the administration rate of administration brought into practice from the other drug administration device; and
a changing step of changing the administration rate of the other device so as to compensate for an insufficient administration rate, when one device between the drug administration device and the other drug administration device can no longer maintain the sharing administration rate.

8. A control method of a drug administration system having first and second drug administration devices which apply a voltage between electrodes so as to percutaneously administer a drug, the control method comprising:
a setting step of setting an administration rate of the drug;
a determination step of determining each sharing administration rate so that the first and second drug administration devices share and realize the administration rate set in the setting step;
a practice step of causing the first and second drug administration devices to practice drug administration using the administration rate determined in the determination step; and
a changing step of changing the administration rate of the other device so as to compensate for an insufficient administration rate, when one device between the first and second drug administration devices can no longer maintain the sharing administration rate determined in the determination step.

9. A control method of a drug administration device which applies a voltage between electrodes so as to percutaneously administer a drug, the control method comprising:
an acquisition step of acquiring a profile in which a drug administration pattern employed by multiple drug administration devices is recorded;
a practice step of causing the drug administration device to practice administration in accordance with the administration pattern which indicates an administration rate and an administration time of the drug, which is recorded in the profile, and which is to be used for the practice of the drug administration device; and
an instruction step of instructing the other drug administration device to follow the administration rate, to start the administration, and to stop the administration in accordance with the administration pattern which is recorded in the profile and which is employed by the other drug administration device.
